# EUROPEAN PATENT APPLICATION

(11) **EP 4 818 164 A1**
(43) Date of publication of application: **07.10.2026**
(21) Application number: 25305489.4
(22) Date of filing: 03.04.2025
(51) Int. Cl.: C07D 401/04, C07D 401/14, A61P 31/14, A61K 31/4439

(54) **2-(PYRIDIN-3-YL)-1H-INDOLE DERIVATIVES AS INHIBITORS OF INTERACTION BETWEEN SPIKE AND ACE2 PROTEINS FOR THE TREATMENT OF RESPIRATORY VIRUS INFECTIONS**

(71) Applicant: Centre National de la Recherche Scientifique, 75016 Paris (FR); Université d'Orléans, 45100 Orléans (FR); Université de Bordeaux, 33000 Bordeaux (FR); Requimte, 4050-453 Porto (PT)
(72) Inventor: PARISSI, Vincent, 33300 Bordeaux (FR); ROUTIER, Sylvain, 45510 Tigy (FR); BURON, Frédéric, 45100 Orléans (FR); SOUSA, Sergio, 4440-752 Valongo (PT); LAPAILLERIE, Delphine, 33600 Pessac (FR); NGO NDIMBA, Alphonsine, 44100 Nantes (FR)
(74) Representative: Novagraaf Technologies

(57) **Abstract**

The present invention relates to an indole derivative of formula (I) 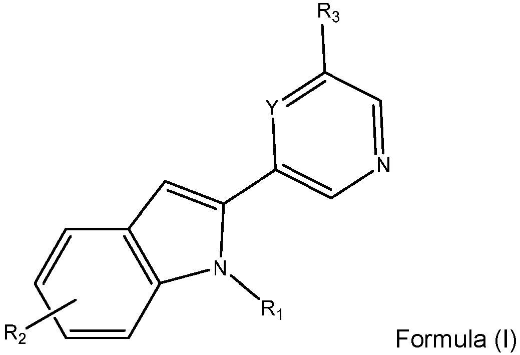 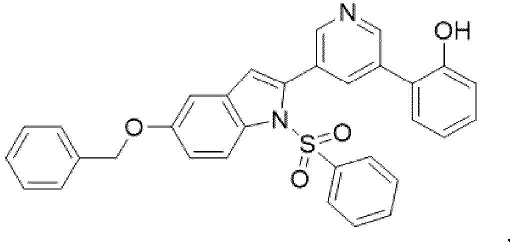 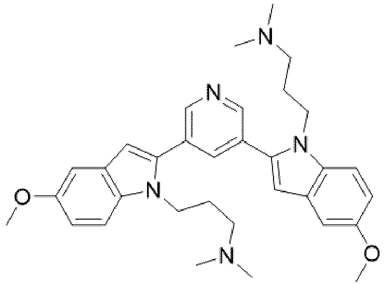 3,3'-(pyridine-3,5-diylbis(5-methoxy-1H-indole-2,1-diyl))bis(N,N-dimethylpropan-1-amine) as inhibitors of interaction between Spike and ACE2 proteins for the treatment of infections due to respiratory viruses, such as e.g. SARS-CoV-1, SARS-CoV-2 or MERS CoV infections.

## Description

### Technical field

The present invention refers to new compounds, a pharmaceutical composition comprising the compound, and to the compound for use as a medicament.

Therefore, the present invention has utility in medical field, especially in the field of treatment of infection diseases.

In the description below, the references into brackets ([ ]) refer to the listing of references situated at the end of the text.

### Background of the Invention

Severe Acute Respiratory Syndrome-coronavirus 2 (SARS-CoV-2) is the positive-strand RNA virus causing agent of severe respiratory syndrome in humans (COVID-19).

Viral entry into cells requires the interaction between the spike S protein of the virus and the Angiotensin-Converting enzyme 2 ACE2 at the surface of the cell membrane. ACE2 has been reported as receptor for SARS-CoV-2 in lung as previously shown for SARS-CoV responsible for the first SARS outbreak in 2003. ACE2 is expressed in human airway epithelia, lung parenchyma, vascular endothelia, kidney cells, and small intestine cells but only weakly detected in the brain. Thus, converging data from the literature indicate the S/ACE2 interaction remains the central entry way for SARS-CoV-2 virus. Being the first contact point between the virus and the cell, the S/ACE2 complex constitute an attractive target for blocking the very early events of the viral infection.

While several vaccines have been developed since the beginning of the pandemic, no efficient drugs have been identified as having strong effect on viral replication usable in clinic, partly due to the lack of understanding of viral replication processes.

Therefore, search for new antiviral approaches remain crucial.

Thus, a need exists of drugs allowing to treat, or at least decrease the symptoms due to SARS-CoV-2 infection. The present invention fulfills these and other needs.

### Description of the invention

The Applicant found, after extensive works, compounds able to inhibit specifically the entry of SARS-COV2 in cells.

Surprisingly, these compounds specifically block the SARS-CoV-2 entry step by impairing the ACE2/S interaction, leading to inhibition of the viral SARS-CoV-2 replication in naturally susceptible cells.

Interestingly, these compounds have similar efficiency on SARS-COV1 virus.

Another advantage of these compounds is they have no or only few cytotoxicity.

Accordingly, in a first aspect, the present invention provides compounds of formula (I) : 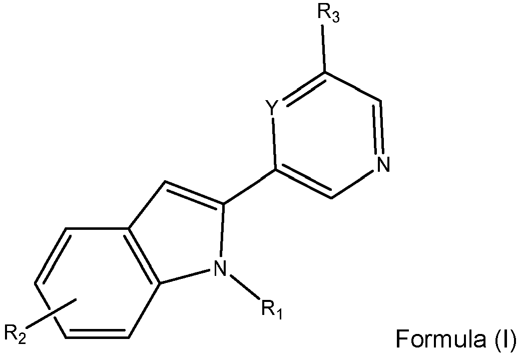 wherein:
- R₁ is selected from the group consisting of:
   C1 to C6 alkyl, C1 to C6 aminoalkyl, C1 to C6 alkoxy, C1 to C6 acyl, and C1 to C6 sulfonyl,
- R₂ is selected from the group consisting of :
   -OH, -OCH₃, -O(CH₂)nOCH₃, with n= 1 or 2, F, -OCH₂O, -OCH₂C₆H₅, -OCH₂C₆H₄F, -O(CH₂)n₁CH₂Rwith n₁ = 2 or 3, R is an aminoalkyl or a cycloaminoalkyl chosen among dimethylamine, diethylamine, piperidine, N-methylpiperazine or morpholine,
- R₃ is selected from the group consisting of :
   - aryl, heteroaryl substituted by -OH, alkoxy, aminoalkyl or halogen, amides, sulfonalides and ureas,
   - Y is selected from the group consisting of -CH and N.

The term "alkyl" refers herein to a linear or branched, saturated, cyclic or aliphatic hydrocarbon group. "C1 to C6 alkyl" is alkyl having from 1 to 6 carbon atoms. Examples of alkyl or C1-C6 alkyl are in particular methyl, ethyl, n-propyl, isopropyl, ethyl isopropyl, n-butyl, isobutyl, sec-butyl, tert-butyl, pentyl, hexyl, heptyl, cyplopropyl, cyclobutyl, cyclopentyl, or cyclohexyl.

The term "aminoalkyl" or "alkylamine" refers herein to an alkyl substituent having an amino group, possibly a primary, a secondary or a tertiary amine, attached to it. Examples of aminoalkyl or C1-C6 aminoalkyl are in particular methylamine, dimethylamine, ethylamine, diethylamine, n-propylamine, isopropylamine, N,N-dimethylpropylamine, n-butylamine, isobutylamine, sec-butylamine, tert-butylamine, n-pentylamine, isoamylamine, n-hexylamine, 2-aminohexane, cyclohexylamine, n-Heptylamine, 2-aminoheptyl, aminomethylcyclopropyl, aminométhylcyclobutyl, aminométhylcyclopentyl, aminométhylcyclohexyl, piperidine, N-methylpiperazine or morpholine.

The term "alkoxy" refers herein to an alkyl group which is singularly bonded to oxygen. "C1-C6 alkoxy" is an alkoxy having from 1 to 6 carbon atoms. Examples of C1-C6 alkoxy are in particular a methoxy, ethoxy, n-propyloxy, isopropyloxy, n-butyloxy, sec-butyloxy, isobutyloxy, tert-butyloxy, n-pentyloxy, isopentyloxy, n-hexyloxy or cyclohexyloxy.

The term "acyl" refers herein to a functional group derived from a carboxylic acid by removing the hydroxyl group from the carboxyl group. Examples of C1-C6 acyl are in particular formyl, acetyl, propionyl, n-butyryl, isobutyryl, n-valeryl, isovaleryl, n-caproyl or cyclohexanecarbonyl.

The term "sulfonyl" refers herein to a functional group formed by a sulphur atom linked to two oxygen atoms by double bonds, and to two other groups or atoms. Examples of sulfonyl groups may be chosen among sulfonic acids, sulfonamides and sulfones. "C1-C6 sulfonyl" is a sulfonyl having from 1 to 6 carbon atoms. Examples of C1-C6 sulfonyl groups are methanesulfonyl, formylsulfonyl, ethanesulfonyl, vinylsulfonyl, n-propylsulfonyl, isopropylsulfonyl, n-butylsulfonyl tert-butylsulfonyl, pentylsulfonyl, cyclopentylsulfonyl, n-hexylsulfonyl or phenylsulfonyl.

The term "aryl" refers herein to a functional group derived from an aromatic ring by removing one hydrogen atom. Examples of aryls are phenyl and its derivatives, such as hydroxyphenyl, phenylethyl, phenylpropyl, butylphenyl, pentylphenyl, hexylphenyl toluyl, naphthyl, biphenyl, or pyridyl.

The term "heteroaryl" (also referred herein as "(Het)Ar") refers herein to a cyclic aromatic ring containing at least one atom other than carbon, such as nitrogen, oxygen, or sulfur. Examples of heteroaryls are pyridyl (i.e. pyridine type heteroaryl), thienyl (i.e. thiophene type heteroaryl), furyl (i.e. furan type heteroaryl), imidazolyl (i.e. imidazole type heteroaryl), indolyl (i.e. indole type heteroaryl), oxazolyl (i.e. oxazole type heteroaryl) and pyrazolyl (i.e. pyrozale type heteroaryl). Preferably, the heteroaryls are chosen among pyridine, indole, thiophene or furane, thiazole or oxazole type.

The term "halogen" refers herein to any of the elements in Group 17 of the periodic table, which includes Fluorine (F), Chlorine (Cl), Bromine (Br) and Iodine (I). Preferably, the halogen is chosen among F and Cl.

The term "amide" refers herein to an organic compound derived from a carboxylic acid, having a nitrogen atom attached to its carbonyl group. Examples of amides are acetamide, benzamide, formamide, N,N-dimethylacetamide, ureide, acylpyrrole, succinimide or phthalimide.

The term "sulfonalide" refers herein to derivatives of sulfonamides that contain both sulfonyl (-SO₂-) and amide (-CONH-) functional groups, with the amide group directly attached to a sulfonyl group, an alkyl, an aryl, a heteroaryl, a functionalized alkyl/aryl or other substituent attached to the sulfonyl group, and an alkyl, an aryl, a cycloalkyl, a heterocycle, or other substituent attached to the carbonyl group of the amide. Examples of sulfonalides are N-(phenylsulfonyl)-acetamide, N-(4-methylphenylsulfonyl)-cyclohexanecarboxamide, or N-(methylsulfonyl)-benzamide.

The term "urea" refers herein to a functional group derived from urea, having the general structure the general structure: -NH-CO-NH-. It may be mono-substituted ureas such as methylurea, or di-substituted ureas such as dimethylurea.

Preferably, R₁ of compound (I) is selected from the group consisting of: 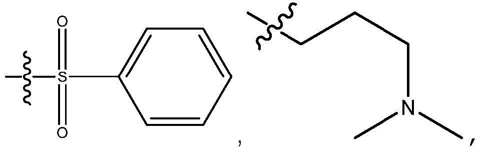 CH₃, ethyl isopropyl, SO₂Me, -COCH₃, -O(CH₂)nOCH₃ with n=1 or 2 and 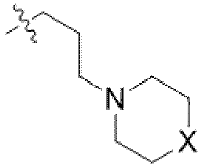 with X is selected from the group consisting of O and NCH₃.

Preferably, R₃ of compound (I) is selected from the group consisting of: 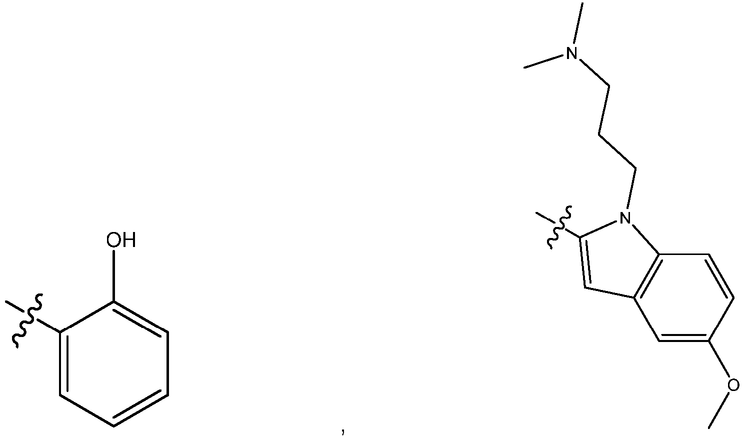 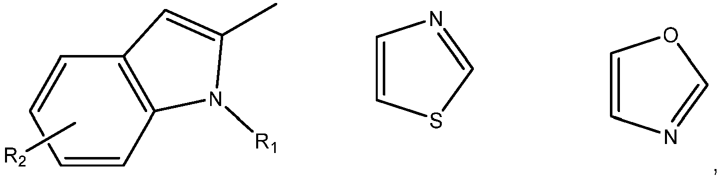 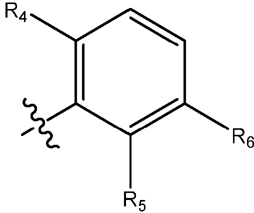 wherein
- R₄ is selected from the group consisting of -OH, F, -CH₂OH, -NHSO₂CH₃, - NHCOCH₃, -NHCONHR and -NHCOOR,
- R₅ is F,
- R₆ is selected from the group consisting of -OCH₃ and isopropyle, 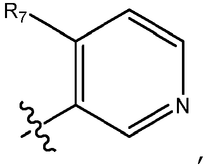 wherein R₇ is selected from the group consisting of -OH and F, 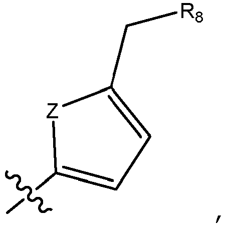 wherein R₈ is selected from the group consisting of -OH, - (CH₂)nN(CH₃)₂, -(CH₂)n-N-methylpiperidine, -(CH₂)n-N-morpholine, with n=0, 1 or 2, and Z is selected from O and S, 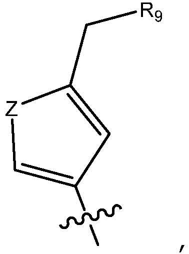 wherein R₉ is selected from the group consisting of -OH, - (CH₂)nN(CH₃)₂, -(CH₂)n-N-methylpiperidine, -(CH₂)n-N-morpholine, with n=0, 1 or 2, and Z is selected from O and S,
   and 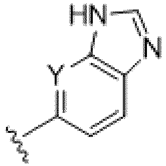 wherein Y is selected from -CH and N.

Advantageously, compound (I) may be selected from the group consisting of: 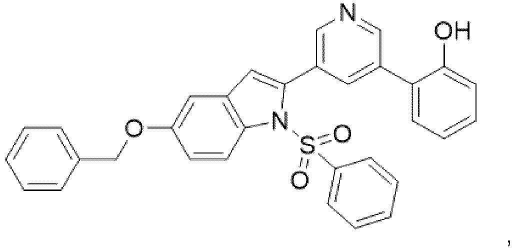 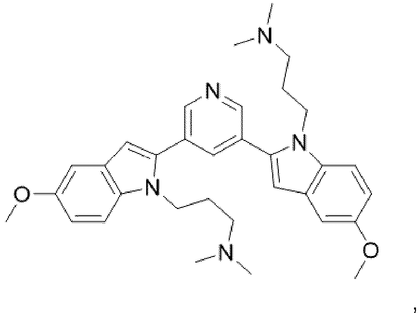 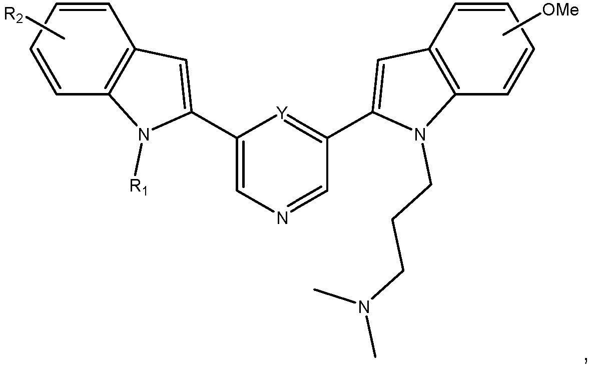 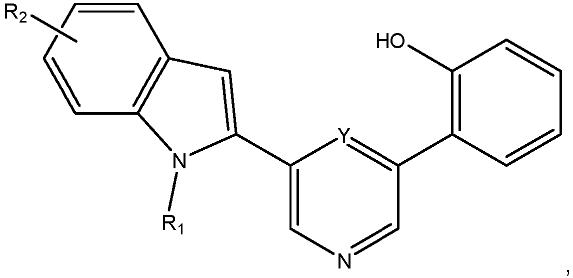 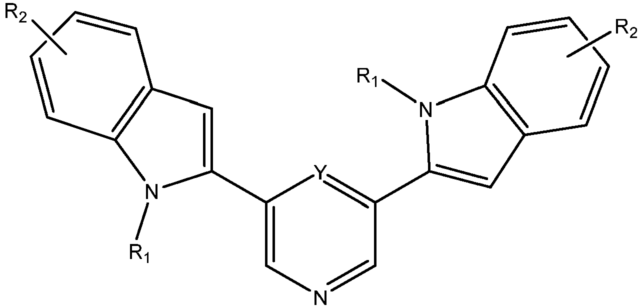 and

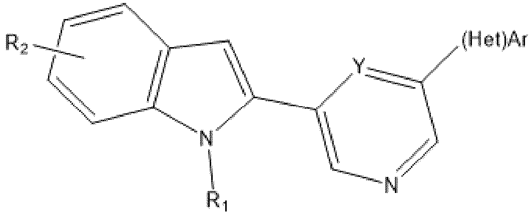

Wherein
- R₁ is selected from the group consisting of CH₃, ethyl propyl, SO₂Me, -COCH₃, - O(CH₂)nOCH₃ with n=2 or 3, 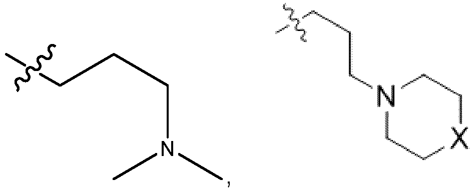 with X is selected from the group consisting of O and -NCH₃,
- R₂ is selected from OH, -OCH₃, -O(CH₂)nOCH₃, with n=1, 2 or 3, F, -OCH₂O, - OCH₂C₆H₅, -OCH₂C₆H₄F, -O(CH₂)n₁CH₂R with n₁ = 2 or 3, R is an aminoalkyl or a cycloaminoalkyl chosen among dimethylamine, diethylamine, piperidine, N-methylpiperazine or morpholine, and
- Y is selected from CH and N.

Whatever the compound of formula (I) described above, R₂ may be preferably located on C5 or on C6 of indole group of formula (I), as shown below:

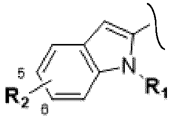

The compounds as defined above may be prepared by methods known in the art, for example in document Jacquemard et al. (Synthesis of 3,5-bis(2-indolyl)pyridine and 3-[(2-indolyl)-5-phenyl]pyridine derivatives as CDK inhibitors and cytotoxic agents. (2008) Bioorg. Med. Chem., 16, 4932-4953 ([1])) and in document Mahboobi et al. (Bis(1H-2-indolyl)methanones as a novel class of inhibitors of the platelet-derived growth factor receptor kinase; J. Med. Chem 2002 45 1002-1018 ([2])), with compound of formula (II) as starting reagent.

In another aspect, the present invention provides the use of a compound of formula (II) 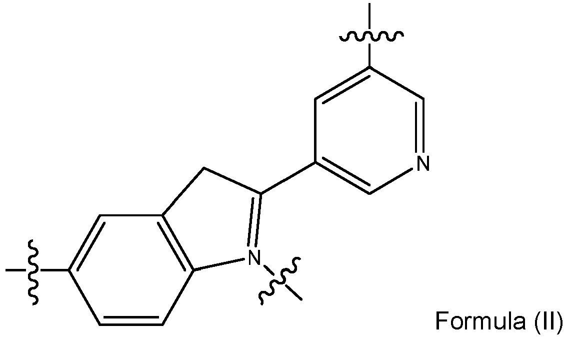 for the preparation of a compound of formula (I) as mentioned above.

The preparation of compounds of formula (I) may be realized for example as described in Examples 1 or 2 below.

In another aspect, the present invention provides a pharmaceutical composition comprising a compound as defined above, or a pharmaceutically acceptable salt thereof, and at least one pharmaceutically acceptable excipient.

The term "pharmaceutically acceptable salt" is meant to include any salt commonly used in the pharmaceutical field and that is adapted to indole derivatives used in the invention according to the general technical knowledge of the skilled person. It can be for example hydrochloride, acetate, phosphate or methanesulfonate, fumarate and oxalate.

The term "pharmaceutical composition" refers herein to a composition comprising at least one active drug and at least one pharmaceutically acceptable excipient, that can be administered to animals and to human beings for the treatment of the disorders and diseases as mentioned above.

According to the invention, one active drug is the compound as defined above. Advantageously, the pharmaceutical composition may comprise at least one other active drug, for example chosen among dexamethasone, remdesivir, azithromycin. The pharmaceutically acceptable excipient may be any substance adapted to be formulated alongside the active ingredient (s) of the pharmaceutical composition of the invention. It may be for example selected among antiadherents, binders, coatings, colours, disintegrants, flavors, glidants, lubricants, preservatives, sorbents, sweeteners, surfactants, lipids, and vehicles.

The pharmaceutical composition may be in any pharmaceutical form, i.e. enteral, parenteral or topical. It may be a medicament for oral administration, for example selected from the group comprising a solution, a syrup, a suspension, an emulsion and oral drops. Alternatively, it may be a medicament in the form of an oral effervescent dosage form, selected from the group comprising tablets, granules, powders. Alternatively, it may be an oral powder or a multiparticulate system, in a form selected from the group comprising beads, granules, mini tablets and micro granules. Alternatively, it may be a medicament in the form of an orodispersible dosage form, selected from the group comprising orodispersible tablets, lyophilised wafers, thin films, a chewable tablet, a tablet and a capsule, a medical chewing gum. Alternatively, it may be a medicament for buccal, intratracheal and sublingual routes, for example selected from the group comprising buccal or sublingual tablets, muco adhesive preparation, lozenges, oro-mucosal drops and sprays. Alternatively, it may be a medicament for topical-transdermal or local administration, for example selected from the group comprising ointments, cream, gel, lotion, patch and foam. Alternatively, it may be a medicament for nasal administration, for example selected from the group comprising nasal drops, nasal spray, nasal powder. Alternatively, it may be a medicament for rectal administration, for example suppository or hard gelatin capsule. Alternatively, it may be a medicament for parenteral administration, for example subcutaneous, intramuscular, intravenous administration.

The pharmaceutical composition of the invention may be prepared using conventional techniques known by the skilled person. For example, the bulk drug substance may be dissolved in a suitable solvent in the presence of one or more of the excipients described above, and mixed.

Advantageously, the pharmaceutical composition may allow delivery of a pharmaceutically acceptable and efficient dose of the compound, for the prevention or the treatment of an infection as defined above. For example, the pharmaceutical composition may comprise a dose of compound of 1 to 50 mg/kg of body weight per day, preferably a dose of 1 to 20 mg/kg of body weight per day. The administration may be carried out with one dose or with a plurality of doses per day.

In another aspect, the present invention provides a compound as defined above, or a pharmaceutically acceptable salt thereof, for use as a medicament.

In another aspect, the present invention provides a compound as defined above, or a pharmaceutically acceptable salt thereof, for use in the prevention or in the treatment of a disease selected from an infection due to respiratory viruses using the cellular ACE2 receptor in their cell entry process.

The infection may be selected in the group of coronaviridae-related infections such as a SARS-CoV-2 infection or a SARS-CoV-1 infection, or a MERS-CoV infection. When the infection is a SARS-CoV-2 infection, SARS-CoV-2 may be the original strain or a circulating variant using the ACE2 cellular receptor for their cell entry pathway, such as alpha variant, beta variant, gamma variant delta variant or omicron variant .

Advantageously, the compound as defined above may inhibit interaction between Spike and ACE2 proteins.

This invention is further illustrated by the following examples with regard to the annexed drawings that should not be construed as limiting.

### Brief description of the figures

- Figure 1 represents: Interaction of AN1-86 with the S-RDB/ACE2 interface (A and B).
- Figure 2 represents: Interaction of AN1-122 with the S-RDB/ACE2 interface (A and B).

### Examples

### Example 1: Method of preparation of a compound of formula (I): compound 5 (AN1-122)

The compound **5** was synthesized as follow. 5-Benzyoxyindole **1** was subjected to a *N*-reaction using the benzenesulfonyl chloride to afford derivative **2** in 93% yield. A regioselective lithiation followed by a stannylation led to the coupling reagent **3.** Derivative **3** and 3,5-dibromopyridine were involved in a mono or di Stille cross coupling reaction . With 1.5 equivalent of **3**, mono reaction occurred in a 30% yield. The monobromo derivative **4** was finally involved in a Suzuki-Myaura reaction to afford **5** (Example 1) in a 10% yield.

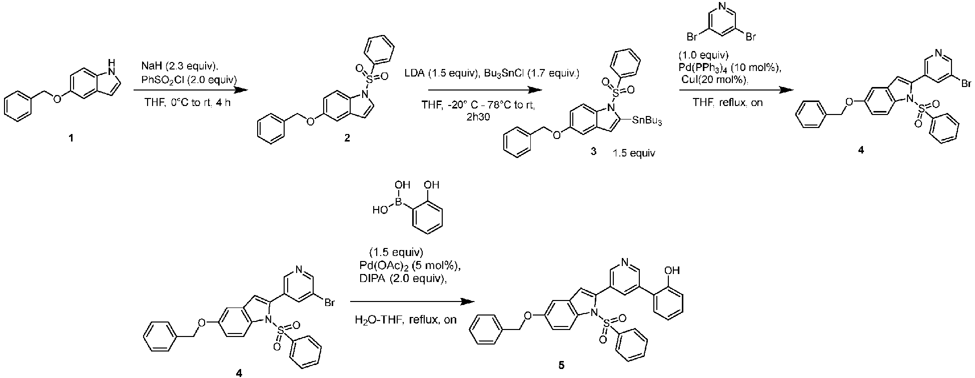

Compound **5** (**AN1-122**) (molecular weight 532.61) is obtained:

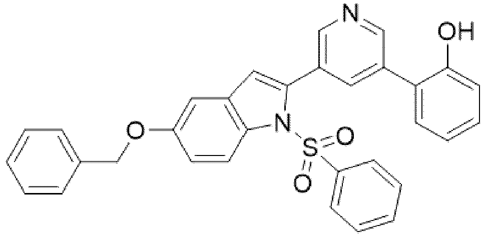

### 5-(Benzyloxy)-1-(phenylsulfonyl)-1H-indole (2)

In a dry flask under argon, a solution of NaH (60% in mineral oil; 2 g, 50 mmol) in dry THF (45 mL) was prepared. The temperature was decreased to 0 °C (ice and water-cooling bath). Then 5-benzyloxyindole (5.582 g, 25 mmol) was dissolved in dry THF (50 mL) and the latter was carefully added to the NaH solution. The reaction mixture was then stirred 30 minutes at room temperature. The temperature was decreased to 0 °C, and benzene sulfonyl chloride (6.4 mL, 30 mmol) was carefully added to the mixture. The reaction mixture was then allowed to warm at room temperature and stirred overnight under argon. The reaction was quenched by pouring the mixture in a beaker of iced water. The target was extracted with ethyl acetate. The organic layer was dried over MgSO₄, filtered and concentrated under reduced pressure. The residue was purified following by recrystallization from ethanol. This step afford the target as white crystals with a yield of 75% (6.81g). Data were in concordance with thus described in Bioorganic & Medicinal Chemistry (2008), 16(9), 4932-4953.

### 5-(Benzyloxy)-1-(phenylsulfonyl)-2-(tributylstannyl)-1H-indole (3).

In a dry flask under argon, the indole derivative **2** (1.82 g, 5.0 mmol) was dissolved in dry THF (15 mL) and the reaction temperature was decreased to -20°C (acetone and dry ice bath). Then, LDA solution (2 M solution in THF; 3.8 mL, 7.5 mmol) was added to the reaction. The mixture was stirred 30 minutes at this temperature. After this time, the temperature was decreased to -78°C (acetone and dry ice bath), and then a solution of Bu₃SnCl in THF (2.3 mL, 8.5 mmol; in 10 mL of THF) was added to the reaction. The cooling bath was then removed and the reaction was stirred and left to return to room temperature over 2 hours. The reaction was quenched by addition of 50 mL of water. The mixture was extracted with ethyl acetate (3*100 mL). The combined organic layers were washed with a saturated solution of potassium fluoride (50 mL) and water (50 mL). The organic layer was dried over MgSO₄, filtered and concentrated under reduced pressure. The target was isolated (3.15 g, 96%, colourless oil) by chromatography on silica gel eluting with a gradient from pure petroleum ether to a mixture constituted of petroleum ether/ethyl acetate: 9/1. Data were in concordance with thus described in Bioorganic & Medicinal Chemistry (2008), 16(9), 4932-4953.

### 5-(Benzyloxy)-2-(5-bromopyridin-3-yl)-1-(phenylsulfonyl)-1H-indole (4)

In a microwave tube, indole derivative **3** (0.652 g, 1 mmol) was dissolved in dry THF (8 mL). The mixture was stirred and purged under argon for 30 minutes. The mixture was heated to 40 °C then 3,5-dibromopyridine (0.249 g, 1.05 mmol), Pd(PPh₃)₄ (0.116 g, 0.1 mmol) and Cul (0.038 g, 0.2 mmol) were added to the mixture. The tube was quickly flushed with argon sealed and stirred at 70 °C overnight. The reaction was then cooled to room temperature and quenched by addition of water (50 mL). The mixture was extracted with ethyl acetate (3*50 mL). The combined organic layers were washed with brine (50 mL), dried over MgSO₄, filtered and concentrated under reduced pressure. The target **3** was isolated (0.127 g, 30%, yellow powder) by chromatography on silica gel eluting with a gradient from pure petroleum ether to pure ethyl acetate. Data were in concordance with thus described in Bioorganic & Medicinal Chemistry (2008), 16(9), 4932-4953.

### 2-(5-(5-(Benzyloxy)-1-(phenylsulfonyl)-1H-indol-2-yl)pyridin-3-yl)phenol (5)

In a microwave tube under argon, 2-phenylboronic acid (0.055 g, 0.4 mmol), indole derivative **4** (0.155 g, 0.26 mmol) and DIPA (0.054 g, 0.53 mmol) were dissolved in THF/H₂O (5/0.5 mL). Both water and THF were purged separately prior to the addition in the previous tube. Pd(OAc)₂ (0.003 g, 0.014 mmol) was then added to the mixture. After this, the tube was flushed with argon, sealed and stirred overnight at reflux. After this time, the reaction was cooled to room temperature and quenched by addition of brine (5 mL). The mixture was extracted with ethyl acetate (3x10 mL). The combined organic layers were dried over MgSO₄ and concentrated under reduced pressure. The residue was purified by column chromatography on silica gel. The target was isolated as a white powder (0.014 g, 10%). Rf (Petroleum ether/Ethyl Acetate: 1/1) = 0.57. ¹H NMR (250 MHz, CDCl₃) δ 8.81 (s, 1H), 8.53 (s, 1H), 8.18 (m, 2H), 7.37 (m, 12H), 6.99 (m, 4H), 6.58 (s, 1H), 5.04 (s, 2H), 2.14 (s, 1H). ¹³C NMR (101 MHz, CDCl₃) δ 156.7 (Cq), 153.8 (Cq), 150.4 (CH), 147.4 (CH), 139.1 (CH), 139.0 (Cq), 138.9 (Cq), 137.0 (Cq), 134.6 (Cq), 134.1 (CH), 133.4 (Cq), 131.6 (Cq), 130.5 (CH), 130.3 (2CH), 129.0 (2CH), 128.8 (2CH), 128.2 (2CH), 127.7 (CH), 126.7 (CH), 124.2 (Cq), 121.2 (CH), 119.9 (Cq), 117.9 (CH), 117.0 (CH), 115.3 (CH), 115.2 (CH), 104.9 (CH), 70.7 (CH₂). HRMS (ESI) m/z: [M + H]+ Calculated for C₃₂H₂₅N₂O₄S 555.134899 ; Found 555.135424.

### Example 2: Method of preparation of a compound of formula (I): compound 11 (AN1-86).

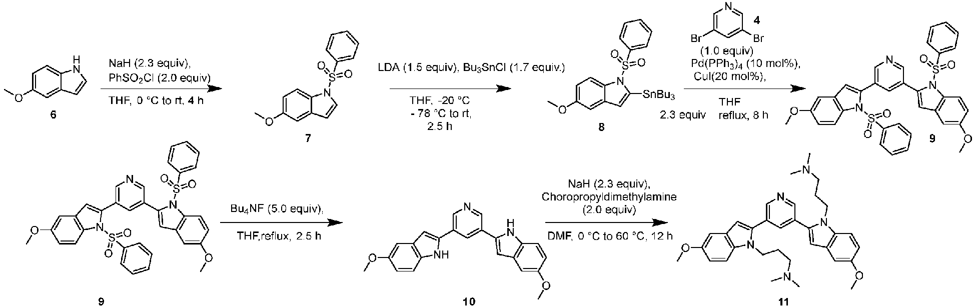

Following a similar procedure as described for **3**, 5-methoxy indole **6** was protected by a benzene sulfonyl group which serve as ortho directed group to perform a regioselective lithiation and stannylation. After these two steps, **8** was isolated and next subjected by a double Stille cross coupling reaction in the presence of 3,5-dibromopyrinine to afford 9 in a 55% yield. A double benzene sulfonyl deprotection afforded 10 which was finally subjected by a double Williamson Alkylation using 3-chloropropyldimethylamine to furnish the attempted derivative **11** (Example 2).

Compound **11** (AN1-86) (molecular weight 539.72) is obtained:

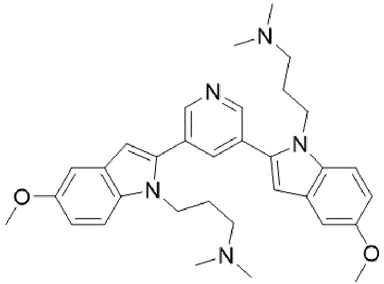

### 5-Methoxy-1-(phenylsulfonyl)-1H-indole (7)

In a dry flask under argon, a solution of NaH (60% in mineral oil; 1.2 g, 30 mmol) in dry THF (45 mL) was prepared. The temperature was decreased to 0 °C (ice and water-cooling bath). Then 5methoxyindole **6** (2.21 g, 15 mmol) was dissolved in dry THF (1.0 M, 15 mL) and the latter was carefully added to the NaH solution. The reaction mixture was then stirred 30 minutes at room temperature. The temperature was decreased to 0 °C, and benzene sulfonyl chloride (3.9 mL, 30 mmol) was carefully added to the mixture. The reaction mixture was then allowed to warm at room temperature and stirred overnight under argon. The reaction was quenched by pouring the mixture in a beaker of iced water. The target was extracted with ethyl acetate. The organic layer was dried over MgSO₄, filtered and concentrated under reduced pressure. The residue was purified by recrystallization from ethanol affording the target as white crystals with a yield of 53%. ¹H NMR (400 MHz, CDCl₃) δ 7.88 (d, J = 9.0 Hz, 1H), 7.86-7.81 (m, 2H), 7.55-7.48 (m, 2H), 7.45-7.38 (m, 2H), 6.97 (d, J = 2.5 Hz, 1H), 6.92 (dd, J = 9.0, 2.5 Hz, 1H), 6.59 (dd, J = 3.5, 0.8 Hz, 1H), 3.81 (s, 3H). HRMS (ESI) m/z: [M + H]+ Calculated for C₁₅H₁₄NO₃S 288.068891 ; Found 288.069001. Mp is in concordance with thus described in Journal of Organic Chemistry 1989, 54, 3264-9.

### 5-Methoxy-1-(phenylsulfonyl)-2-(tributylstannyl)-1H-indole (8)

In a dry flask under argon, the indole derivative **7** (1.15 g, 4.0 mmol) was dissolved in dry THF (10 mL) and the reaction temperature was decreased to -20°C (acetone and dry ice bath). Then, LDA solution (2 M solution in THF; 3.0 mL, 6.0 mmol) was added to the reaction. The mixture was stirred 30 minutes at this temperature. After this time, the temperature was decreased to -78 °C (acetone and dry ice bath), and then a solution of Bu₃SnCl in THF (2 mL, 6.0 mmol; in 10 mL of THF) was added to the reaction. The cooling bath was then removed and the reaction was stirred and left to return to room temperature over 2 hours. The reaction was quenched by addition of 50mL of water. The mixture was extracted with ethyl acetate (3*100 mL). The combined organic layers were washed with a saturated solution of potassium fluoride (50 mL) and water (50 mL). The organic layer was dried over MgSO₄, filtered and concentrated under reduced pressure. The target was isolated (1.88 g, 83%, colourless oil) by chromatography on silica gel eluting with a gradient from pure petroleum ether to a mixture constituted of petroleum ether/ethyl acetate: 9/1. Rf (Petroleum ether/ethyl acetate : 95/5): 0.75. ¹H NMR (400 MHz, CDCl₃) δ 7.73 (d, *J* = 9.1 Hz, 1H), 7.62 (d, *J* = 7.7 Hz, 2H), 7.53-7.45 (m, 1H), 7.38 (t, *J* = 8.0 Hz, 2H), 6.96 (d, *J* = 2.5 Hz, 1H), 6.82 (dd, *J* = 8.9, 1.9 Hz, 1H), 6.77 (s, 1H), 3.80 (s, 3H, OCH₃), 1.68-1.48 (m, *J* = 8.6, 7.9 Hz, 6H), 1.36 (h, *J* = 7.3 Hz, 6H), 1.28-1.10 (m, 6H), 0.91 (t, *J* = 7.3 Hz, 9H). ¹³C NMR (101 MHz, CDCl₃) δ 156.4 (Cq), 144.7(Cq), 139.5 (Cq), 133.4 (Cq), 133.4 (CH), 133.1 (Cq), 129.2 (2CH), 126.3 (2CH), 120.7 (CH), 114.5 (CH), 113.1( CH), 102.6 (CH), 55.7 (OCH₃), 29.1 (3CH₂), 27.5 (3CH₂), 13.8 (3CH₃), 11.9 (3CH₂). HRMS (ESI) m/z: [M + H]+ Calculated for C₂₇H₄₀NO₃SSn 578.174536 ; Found 578.174515.

### 3,5-Bis(5-methoxy-1-(phenylsulfonyl)-1H-indol-2-yl)pyridine (9)

In a microwave tube, indole derivative **8** (2.53 g, 4.4 mmol) was dissolved in dry THF (20 mL). The mixture was stirred and purged under argon for 30 minutes. The mixture was heated to 40 °C then 3,5dibromopyridine (0.452 g, 1.9 mmol), Pd(PPh₃)₄ (0.219 g, 0.19 mmol) and Cul (0.072 g, 0.038 mmol) were added to the mixture. The tube was quickly flushed with argon sealed and stirred at 70 °C overnight. The reaction was then cooled to room temperature and quenched by addition of water (50 mL). The mixture was extracted with ethyl acetate (3*50 mL). The combined organic layers were washed with brine (50 mL), dried over MgSO₄, filtered and concentrated under reduced pressure. The target was isolated (0.69 g, 55%, yellow powder) by chromatography on silica gel eluting with a gradient from pure petroleum ether to pure ethyl acetate. Rf (Petroleum ether/ ethyl acetate: 1/1 ): 0.68. ¹H NMR (400 MHz, CDCl₃) δ 8.75 (d, *J* = 2.1 Hz, 2H), 8.21 (d, *J* = 9.1 Hz, 2H, 2xCH), 8.15 (t, *J =* 2.1 Hz, 1H), 7.48-7.42 (m, 6H, 6xCH), 7.30 (t, *J* = 7.8 Hz, 4H, 4xCH), 7.01 (dd, *J* = 9.1, 2.5 Hz, 2H, 2xCH), 6.92 (d, *J* = 2.5 Hz, 2H, 2xCH), 6.68 (s, 2H, 2xCH), 3.83 (s, 6H, 2xCH₃, OCH₃). ¹³C NMR (101 MHz, CDCl₃) δ 157.5 (2Cq), 150.0 (2CH), 138.8 (2CH), 138.6 (2Cq), 136.8 (2Cq), 134.0 (CH), 133.2 (2Cq), 131.8 (2Cq), 129.1 (4CH), 127.8 (2Cq), 126.9 (4CH), 117.8(2CH), 116.0(2CH), 114.6(2CH), 103.6(2CH), 55.8(2OCH₃). HRMS (ESI) m/z: [M + H]+ Calculated for C₃₅H₂₈N₃O₆S₂ 650.141404 ; Found 650.140741.

### 3,5-Bis(5-methoxy-1H-indol-2-yl)pyridine (10)

In a microwave tube, indole derivative **9** (0.1 g, 0.12 mmol) was dissolved in dry THF (5 mL). TBAF solution (1 M in THF; 0.6 mL, 0.6 mmol) was added to the mixture and the reaction was stirred heated to 70 °C for 150 minutes at this temperature. After this time the solution became red, knowing it started yellow. The reaction was then cooled to room temperature and concentrated under reduced pressure. Water was added to the residue and was extracted with dichloromethane (3*10 mL). The combined organic layers were washed with brine (10 mL), dried over MgSO₄, filtered and concentrated under reduced pressure. The target was isolated (0.067 g, 99%, yellow powder) by chromatography on silica gel eluting with a gradient from pure petroleum ether to pure ethyl acetate. Rf (Petroleum ether/ Ethyl acetate : 1/1 ) : 0.35. ¹H NMR (250 MHz, DMSO-*d₆*) δ 11.54 (s, 2H, 2xNH), 8.59 (s, 1H), 7.66-7.43 (m, 2H, 2xCH), 7.31 (d, *J* = 8.8 Hz, 2H, 2xCH), 7.05 (d, *J =* 2.4 Hz, 2H, 2xCH), 7.01 (s, 2H, 2xCH), 6.77 (dd, *J* = 8.8, 2.4 Hz, 2H, 2xCH), 3.74 (s, 6H, 2xCH₃, OCH₃). ¹³C NMR (101 MHz, DMSO-*d₆*) δ 153.8 (2Cq), 144.3 (2Cq), 134.9 (2Cq), 132.6 (2Cq), 134.0 (2CH), 131.5 (CH), 131.4 (2CH), 128.8 (2Cq), 128.8 (2CH), 128.6 (2CH), 127.6 (2CH), 112.6 (2CH), 112.1(2CH), 101.7 (2CH), 100.1 (2CH), 55.3 (2OCH₃).

### 3,3'-(Pyridine-3,5-diylbis(5-methoxy-1H-indole-2,1-diyl))bis(N,N-dimethylpropan-1-amine) (11)

In a microwave tube under argon, indole derivative **10** (0.11 g, 0.29 mmol) was dissolved in dry DMF (10 mL). The temperature of the reaction was decreased to 0 °C (water and ice bath), the NaH (60% - in mineral oil; 0.23 g, 0.59 mmol) was added to the reaction. The mixture was stirred 30 minutes at this temperature. A change in the colour was observed after addition of NaH, a red solution was obtained. After this time, a solution of 3-chloropropyldimethylamine (0.71 g, 0.59 mmol) in DMF (5 mL) was added to the reaction. The mixture was then heated to 60°C overnight. After this time, the reaction was cooled to room temperature and quenched by addition of water (10 mL). The mixture was extracted with dichloromethane (3*10 mL), dried over MgSO₄, filtered and concentrated under reduced pressure. The residue was purified by column chromatography on silica gel eluting with a gradient dichloromethane/trimethylamine: 1/0.1 to a mixture dichloromethane/methanol/trimethylamine: 9/1/0.1. The target was obtained as a yellow oil (0.104 g, 67%). Rf (CH₂Cl₂/ MeOH: 9/1): 0.31. ¹H NMR (250 MHz, CDCl₃) δ 8.78 (d, *J* = 2.1 Hz, 2H, 2xCH), 7.94 (t, *J* = 2.1 Hz, 1H, CH), 7.35 (d, *J* = 8.9 Hz, 2H, 2xCH), 7.11 (d, *J* = 2.5 Hz, 2H, 2xCH), 6.94 (dd, *J* = 8.9, 2.5 Hz, 2H, 2xCH), 6.58 (d, *J* = 0.8 Hz, 2H, 2xCH), 4.31-4.20 (m, 4H, 2xCH₂), 3.88 (s, 6H, 2xCH₃), 2.17 (t, *J* = 6.8 Hz, 4H, 2xCH₂), 2.11 (s, 12H, 2xCH₃), 1.84 (p, *J* = 6.9 Hz, 4H, 2xCH₂). ¹³C NMR (101 MHz, CDCl₃) δ 154.6 (2Cq), 148.7 (2CH), 137.4 (2Cq), 136.4 (CH), 133.4 (2Cq), 129.2 (2Cq), 128.5 (2Cq), 113.0 (2CH), 111.1 (2CH), 103.7 (2CH), 102.4 (2CH), 56.4 (2CH₂), 55.9 (2OCH₃), 45.1 (4CH₃), 42.3 (2CH₂), 28.0 (2CH₂). HRMS (ESI) m/z: [M + H]+ Calculated for C₃₃H₄₂N₅O₂ 540.333302 ; Found 540.333345.

### Example 3: effects of Compound AN1-122 and compound AN1-86 as new antiviral drugs targeting multiple SARS-CoV S/ACE2 interfaces

### Computational analysis of the interaction of AN1-86 and AN1-122 with the S-RDB/ACE2 interface

By using computational studies, it is possible to better understand the binding of AN1-86 and AN1-122 to the target protein. The first step was to perform molecular docking of both compounds into the target protein. AN1-86 was predicted to be a better binder to both the S-RDB/ACE2 complex and the isolated S-RDB, with scores of 84.88 and 73.49, respectively. As for AN1-122, it presented a score of 81.19 when docked into the S-RDB/ACE2 complex and 72.50 when docked into S-RDB alone.

The next step was to perform MD simulations of our protein/ligand complexes. For each complex, three different 500 ns replicas were performed. First, the simulations of AN1-86 in complex with S-RDB showed that the ligand remained in complex at the beginning of the simulation, but it started to leave the binding pocket after 300 ns of simulation. This indicates that, while it can transiently bind the S-RDB, it does not strongly bind to the spike protein. As for AN1-122, this molecule starts to leave the binding pocket after 100 ns of simulation, indicating a weaker interaction with isolated S-RDB.

When considering the binding of the AN1-86 and AN1-122 to the S-RDB/ACE2 interface, both compounds remain in the binding pocket across all three replicas. Furthermore, RMSD calculations indicate that the system remains stable throughout the simulation. The Solvent Accessible Surface Area (SASA) calculations (Table 2) show that AN1-86 and AN1-122 have 81.64 ± 0.05 % and 91.87 ± 1.09 % of their surface buried in the binding pocket. This indicates that both compounds are buried in the binding pocket over the course of the simulation, with AN1-122 having a higher percentage of its surface buried in its binding pocket due to its more hydrophobic properties.

**Table 2 - Average SASA values for AN1-86 and AN1-122 in complex with the S-RDB/ACE2 interface considering three replicas for each system.**

| Ligand | Average SASA (Å²) | % of Ligand Buried |
|---|---|---|
| AN1-86 | 157.4 ± 44.3 | 82.3 ± 4.7 |
| AN1-122 | 62.9 ± 17.3 | 91.9 ± 2.3 |

After establishing that both ligands are stably bound to the S-RDB/ACE2 interface, the next step was to perform free energy calculations. This work used the MM-PBSA and MM-GBSA methods, with the results available in Tables 3 and 4.

**Table 3 - Calculation of the free energy of binding for AN1-86 and AN1-122 in complex with the S-RDB/ACE2 interface using the MM-PBSA method.**

| Ligand | ΔG_{binding} (kcal/mol) | ΔEₑₗₑ (kcal/mol) | ΔE_{vdw} (kcal/mol) | ΔE_{PB} (kcal/mol) | ΔE_{non-Polar} (kcal/mol) | ΔE_{Disper} (kcal/mol) |
|---|---|---|---|---|---|---|
| AN1-86 | -23.3 ± 2.3 | -116.4 ± 1.7 | -67.9 ± 3.4 | 122.5 ± 1.9 | -46.6 ± 1.3 | 88.4 ± 3.1 |
| AN1-122 | -18.5 ± 1.6 | -7.1 ± 0.4 | -63.8 ± 2.2 | 16.9 ± 0.8 | -44.7 ± 0.6 | 80.2 ± 1.1 |

**Table 4 - Calculation of the free energy of binding for AN1-86 and AN1-122 in complex with the S-RDB/ACE2 interface using the MM-GBSA method.**

| Ligand | ΔG_{binding} (kcal/mol) | ΔEₑₗₑ (kcal/mol) | ΔE_{vdw} (kcal/mol) | ΔE_{GB} (kcal/mol) | ΔE_{surf} (kcal/mol) |
|---|---|---|---|---|---|
| AN 1-86 | -47.1 ± 3.6 | -465.5 ± 6.8 | -67.9 ± 3.4 | 495.2 ± 6.3 | -8.7 ± 0.4 |
| AN1-122 | -41.7 ± 3.4 | -28.4 ± 1.5 | -63.8 ± 2.2 | 58.9 ± 3.4 | -8.4 ± 0.2 |

Both MM-PBSA and MM-GBSA methods show that AN1-86 and AN1-122 have high affinities towards the S-RDB/ACE2 interface. Furthermore, the results indicate that AN1-86 is the most potent inhibitor. A per-residue decomposition of the results was implemented to better understand the interaction between these compounds and the S-RDB/ACE2 interface. The results for both ligands and both methods are displayed in Table 5.

**Table 5 - Residues that contribute the most to the affinity of AN1-86 and AN1-122 towards the S-RDB/ACE2 interface, according to the MM-PBSA and MM-GBSA methods.**

| Method | AN1-86 | AN1-122 |
|---|---|---|
| MM-PBSA | ACE2: Leu29, Asp30, His34, Glu37, Thr92, Gln96, Pro389. | ACE2: Leu29, Asp30, Asn33, His34, Pro389, Arg393. |
| | S-RDB: Arg403, Asp405, Glu406, Tyr505. | S-RDB: Arg403, Tyr495, Tyr505. |
| MM-GBSA | ACE2: Leu29, His34, Thr92, Gln96, Pro389, Arg393. | ACE2: Leu29, Asp30, Asn33, His34, Pro389. |
| | S-RDB: Arg403, Asp405, Glu406, Tyr495, Tyr505 | S-RDB: Arg403, Tyr495, Tyr505. |

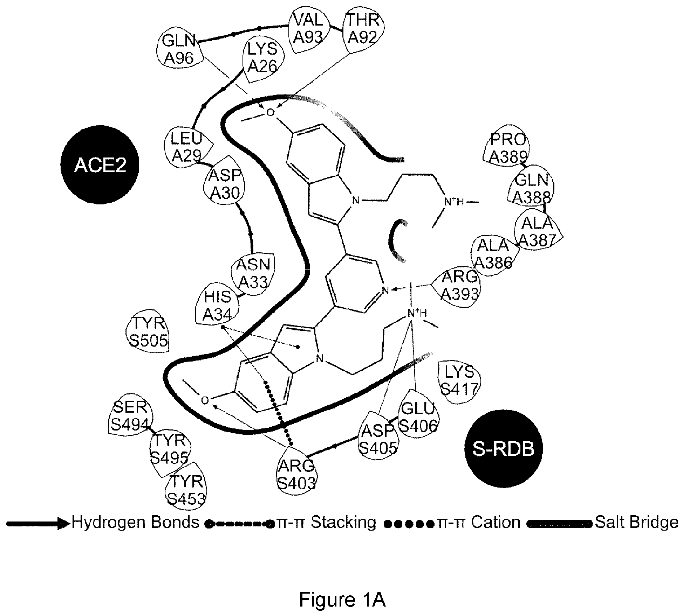

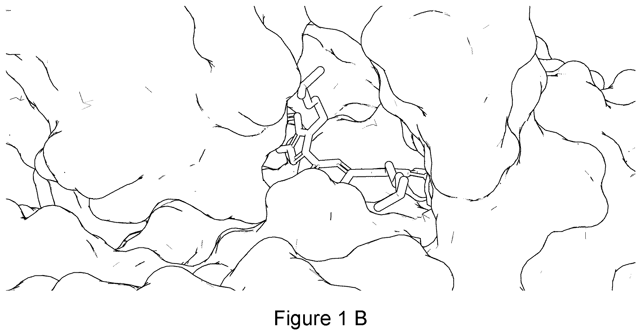

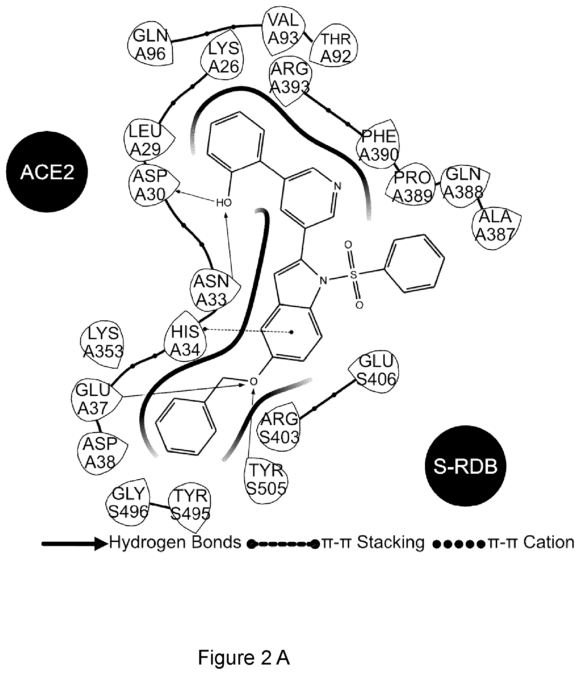

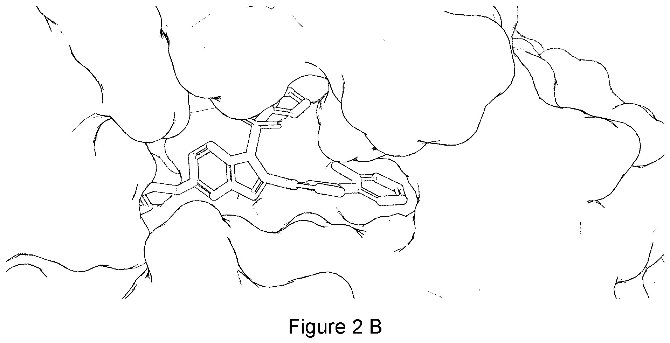

Amongst the contributing residues, the ones that have the most impact in the interaction with AN1-86 are: ACE2-His34, ACE2-Glu37, ACE2-Pro389, S-RDB-Asp405, S-RDB-Glu406 and S-RDB-Tyr505. As for the interaction with AN1-122, these are the most contributing residues: ACE2-Asp30, ACE2-Asn33, ACE2-His34, S-RDB-Arg403 and S-RDB-Tyr505. Additionally, the N-glycosylated Asn90 from ACE2 also greatly impacts the affinity of both ligands towards the S-RDB/ACE2 interface. In the case of AN1-86, this group stays more open and interacts with one of the protonated tertiary amine groups via hydrogen bonds. As for AN1-122, the glycosylated group closes around the phenylsulfonyl group, forming a hydrophobic pocket for this group. The mode of interaction of each ligand with the protein was also studied and can be seen in Figures 1 and 2.

Over the course of the MD simulation, AN-86 performs multiple interactions. As previously mentioned, one of the protonated tertiary amines interacts with the N-glycosylated group via hydrogen bonds. Asp405 and Glu406 of S-RDB establish salt bridges with the other protonated tertiary amine. Moreover, ACE2 Thr92 and Gln96 form hydrogen bonds with one of the terminal methoxy groups, while S-RDB Arg403 interacts similarly with the other terminal methoxy group. This residue also establishes a π-cation interaction with one of the aromatic rings from the indole group. One more hydrogen bond can be seen as the nitrogen from the pyridine group acts as a hydrogen bond acceptor for an interaction with ACE2 Arg393. Finally, ACE2 His34 interacts with both rings from the indole group via π-π stacking interactions.

In the case of AN-122, the oxygen in the ether bridge is a hydrogen bond acceptor for interactions with ACE2 Glu37 and with S-RDB Thr505. The terminal hydroxyl group acts as a hydrogen bond donor in the interaction with ACE2 Asp30. Furthermore, this hydroxyl group also acts as a hydrogen bond acceptor in the interaction with ACE2 Asn33. Finally, ACE2 His34 can establish π-π stacking interactions with the central indole group.

The previously described studies indicate that both compounds can bind and perform stable interactions with the S-RDB/ACE2 interface. Furthermore, the results suggest that one of the compounds is a more robust binder than the other. AN1-86 presents a more negative predicted binding free energy using both MM-PBSA and MM-GBSA methods. Additionally, the interaction analysis shows that this compound establishes stronger interactions than AN1-122. Therefore, it can be concluded that while both compounds are able to bind to the S-RDB/ACE2 interface, AN1-86 is predicted to be the most potent binder.

The docking modelisation showed that when the simulations are done only with the spike protein, the drugs bind weakly and in the MD simulations quickly exit to the solvent. In contrast, when the simulations are done with the complex ACE2-Spike the drug remain stably bound to the complex. Moreover, when we analyzed the contribution to different amino acid residues of the interface to the binding of the drugs, we can observe that the amino acid residues in ACE2 are the major contributors to drug binding, representing 60-75% of the drugs binding free energy. Taken together these data suggest that the drugs efficiently target the ACE2 cellular receptor and parallel finely with the biochemical analyses. These data are thus consistent with the mode of action of the compounds which binds the ACE2 receptor and prevent Spike binding and subsequent viral entry. The selected drugs thus constitute one of the most promising therapeutical agents specifically targeting this early replication step of coronaviruses and is a current candidate for preclinical trials.

### REFERENCES

1. Jacquemard et al. Synthesis of 3,5-bis(2-indolyl)pyridine and 3-[(2-indolyl)-5-phenyl]pyridine derivatives as CDK inhibitors and cytotoxic agents. (2008) Bioorg. Med. Chem., 16, 4932-4953.
2. Mahboobi et al. (Bis(1H-2-indolyl)methanones as a novel class of inhibitors of the platelet-derived growth factor receptor kinase; J. Med. Chem 2002 45 1002-1018.

## Claims

1. Compound of formula (I) : 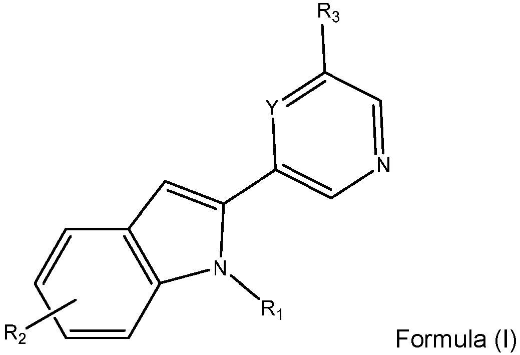 wherein:
- R₁ is selected from the group consisting of:
C1 to C6 alkyl, C1 to C6 aminoalkyl, C1 to C6 alkoxy, C1 to C6 Acyl, and C1 to C6 sulfonyl,
- R₂ is selected from the group consisting of :
-OH, -OCH₃, -O(CH₂)nOCH₃, with n=2 or 3, F, -OCH₂O, -OCH₂C₆H₅, -OCH₂C₆H₄F, -O(CH₂)n₁CH₂R with n₁ = 2 or 3, R is an aminoalkyl or a cycloaminoalkyl chosen among dimethylamine, diethylamine, piperidine, N-methylpiperazine or morpholine,
- R₃ is selected from the group consisting of :
- aryl, heteroaryl substituted by OH, alkoxy, aminoalkyl or halogen, amide, sulfonamide and urea,
- Y is selected from the group consisting of CH and N.

2. The compound according to claim 1, wherein:
- R₁ is selected from the group consisting of: 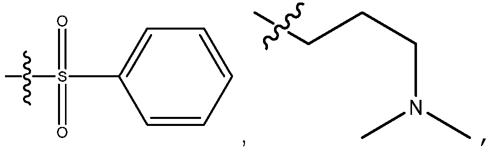 -CH₃, ethyl isopropyl , -SO₂Me, - COCH₃, -O(CH₂)nOCH₃ with n= 2or 3, and 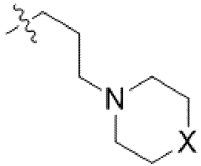 with X is selected from the group consisting of O and -NCH₃.

3. The compound according to claim 1 or 2, wherein heteroaryl of R₃ is selected among pyridine, indole, thiophene or furane, thiazole and oxazole type.

4. The compound according to claim 1 or 2, wherein:
- R₃ is selected from the group consisting of: 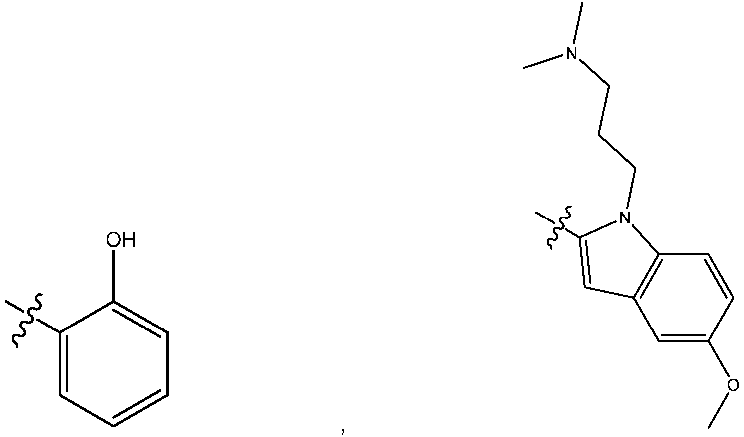 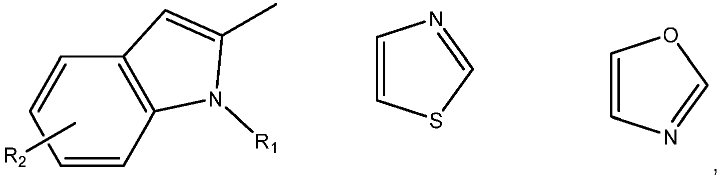 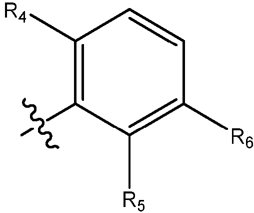 wherein
- R₄ is selected from the group consisting of -OH, F, -CH₂OH, -NHSO₂CH₃, - NHCOCH₃, -NHCONHR and -NHCOOR,
- R₅ is F,
- R₆ is selected from the group consisting of OCH₃ and isopropyle, 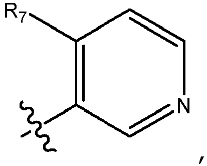 wherein R₇ is selected from the group consisting of -OH and F, 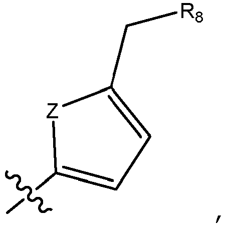 wherein R₈ is selected from the group consisting of -OH, - (CH₂)nN(CH₃)₂, -(CH₂)n-N-methylpiperidine, -(CH₂)n-N-morpholine, with n=0, 1 or 2, and Z is selected from O and S, 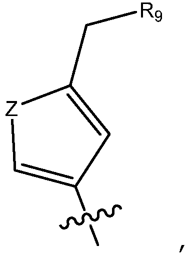 wherein R₉ is selected from the group consisting of -OH, - (CH₂)nN(CH₃)₂, -(CH₂)n-N-methylpiperidine, -(CH₂)n-N-morpholine, with n=0, 1 or 2, and Z is selected from O and S,
and 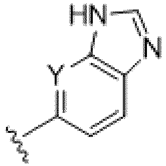 wherein Y is selected from -CH and N.

5. The compound according to any one of the preceding claims, which is selected from the group consisting of: 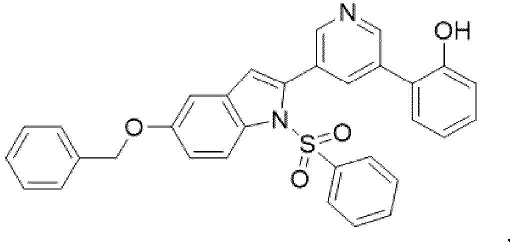 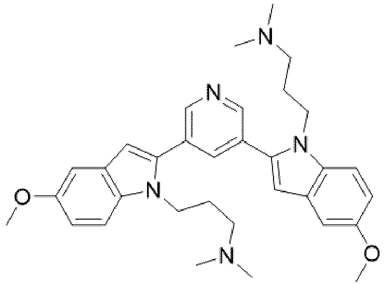 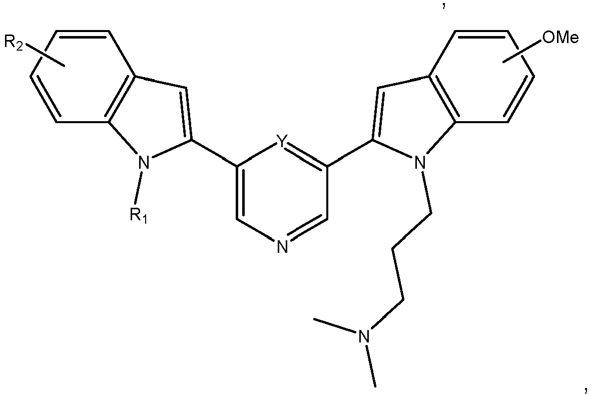 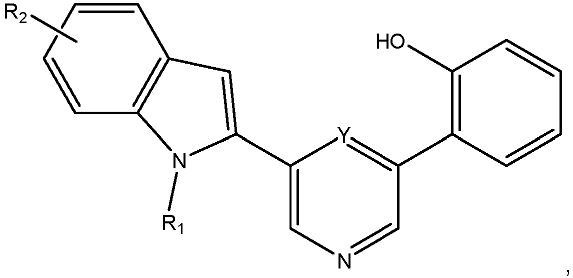 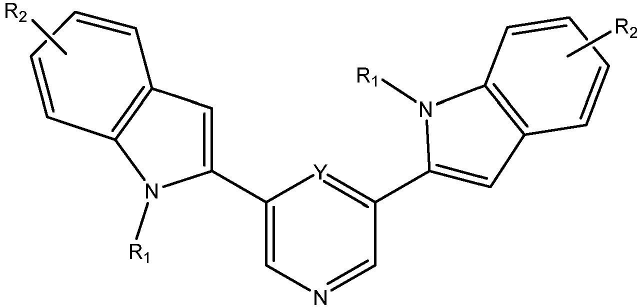 and 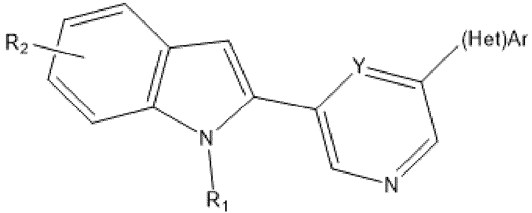 Wherein
- R₁ is selected from the group consisting of -CH₃, -Et IPr, -SO₂Me, -COCH₃, O(CH₂)nOCH₃ with n=0, 1, or 2 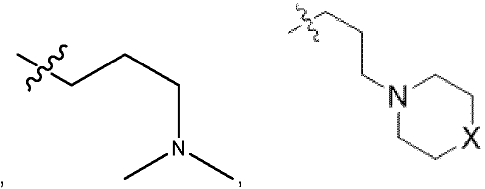 with X is selected from the group consisting of O and -NCH₃,
- R₂ is selected from the group consisting of -OH, -OCH₃, -O(CH₂)nOCH₃, with n=0, 1 or 2, F, -OCH₂O, -OCH₂C₆H₅, -OCH₂C₆H₄F, -O(CH₂)n₁CH₂Rn₂ with n₁ = 1 or 3, R is an aminoalkyl or a cycloaminoalkyl chosen among dimethylamine, diethylamine, piperidine, N-methylpiperazine or morpholine,
- Y is selected from -CH and N.

6. The compound according to any one of the preceding claims, wherein R₂ is located on C5 or on C6 of indole group of formula (I).

7. A pharmaceutical composition comprising a compound as defined in any one of claims 1 to 5, or a pharmaceutically acceptable salt thereof, and at least one pharmaceutically acceptable excipient.

8. The pharmaceutical composition according to claim 7, which is in an enteral, parenteral or topical form.

9. A compound as defined in any one of claims 1 to 6, or a pharmaceutically acceptable salt thereof, for use as a medicament.

10. A compound as defined in any one of claims 1 to 6, or a pharmaceutically acceptable salt thereof, for use in the prevention or in the treatment of an infection due to respiratory viruses using the cellular ACE2 receptor in their cell entry process.

11. The compound for use according to claim 10, wherein the infection is selected in the group of coronaviridae-related infections, such as a SARS-CoV-2 infection or a SARS-CoV-1 infection, and a MERS CoV infection.

12. The compound for use according to claim 10, wherein SARS-CoV-2 is the original strain or a circulating variant using the ACE2 cellular receptor for their cell entry pathway, such as alpha variant, beta variant, gamma variant delta variant or omicron variant.

13. The compound for use according to claim 11 or 12, for inhibiting interaction between Spike and ACE2 proteins.

14. Use of a compound of formula (II) 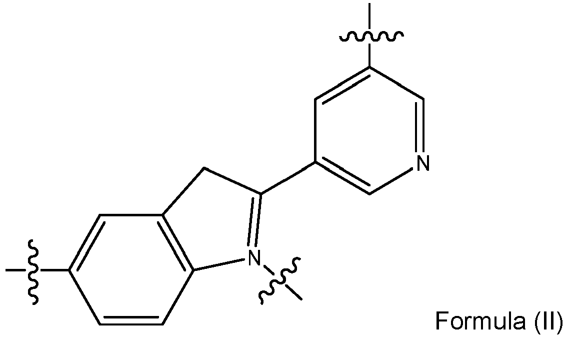 for the preparation of a compound of formula (I) as mentioned in any one of claims 1 to 6.
